(19) Europäisches Patentamt
European Patent Office
Office européen des brevets

(11) Publication number: **0 165 912 B1**

(12) **EUROPEAN PATENT SPECIFICATION**

(45) Date of publication of patent specification: **07.02.90**

(51) Int. Cl.⁵: **B 01 J 20/32,** B 01 D 15/00

(21) Application number: **85850148.9**

(22) Date of filing: **29.04.85**

(54) **Thioether adsorbent intended for the separation of proteins and the like.**

(30) Priority: **17.05.84 SE 8402662**

(43) Date of publication of application: **27.12.85 Bulletin 85/52**

(45) Publication of the grant of the patent: **07.02.90 Bulletin 90/06**

(84) Designated Contracting States: **DE FR GB**

(56) References cited:
**GB-A-1 544 867**
**GB-A-2 006 640**
**GB-A-2 092 470**
**US-A-3 917 527**

**Makromol Chem., Macromol Symp. 17, 359 (1988)**

**The file contains technical information submitted after the application was filed and not included in this specification**

(73) Proprietor: **Porath, Jerker**
**Bodalsvägen 4A**
**S-18136 Lidingö (SE)**

(72) Inventor: **Porath, Jerker**
**Bodalsvägen 4A**
**S-18136 Lidingö (SE)**

(74) Representative: **Rosenquist, Per Olof et al**
**P.O. Rosenquist Patentbyra AB Storgatan 35**
**S-151 36 Södertälje (SE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Different kinds of adsorbents are used within the fields of biochemistry and biotechnology for the isolation of macro- as well as "micromolecules" and for the immobilization of e.g. enzymes for technical applications, as well as antigens and antibodies for the diagnosis of diseases. The present invention belongs to this category of adsorbents but distinguishes from conventional types of adsorption agents as to certain characteristic properties.

The US—A—3 917 527 concerns adsorbents for separation and purification of biomolecules such as peptides, vitamins, hormones, proteins, membranes, cells and the like by means of charomatographic adsorbents. The adsorbents consists of a solid, water-insoluble matrix of polyacrylamide, agarose and the like with attached hydrocarbon arms, covalently bound to the matrix through a difunctional linking group such as NH, S, and terminated in a functional group such as $NH_2$, SH, $SO_3H$.

Our invention relates to a finely divided adsorbent consisting of particles having in a particularly useful form a spherical configuration with a diameter in the magnitude of 1—1000 µm. The particles are distinguished by their structure of a polymeric netting penetrating the entire particles or acting as a surface coating, and containing substituents having the structure

$$-X-\overset{|}{\underset{|}{C}}-CH_2-Y- \quad \text{or} \quad -X-\overset{|}{C}=CH-Y- \qquad (I)$$

wherein X can be $>SO_2$, $>SO$, $>S$ or $>Se$ and Y is $>S$ or NR, for example

$$R-X-CH_2-CH_2-S- \qquad (II)$$

and

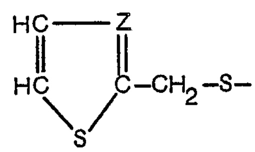

or

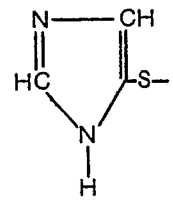

(III)

wherein Z is N or CH, and R can be an arbitrary substituent.

A distinguishing feature of the invention is its adsorption properties, the nature of which can be of a kind similar to that of so-called hydrophobic adsorbents. For example, the adsorption of proteins is increased in the presence of high concentrations of aqueous structure organized salts such as e.g. alkali chlorides, alkali sulfates and magnesium sulfates. Apparently however, adsorbents according to the invention preferably adsorb other proteins than is the case with hydrophobic adsorbents based on the presence of alkyl groups containing e.g. 8—18 carbon atoms.

An unexpected and surprising discovery is that R can be hydrophilic, e.g. $-CH_2-CH_2-OH$, or even $-CH_2-CHOH-CH_2OH$, which indicates that a characteristic and hitherto unknown adsorption effect is connected with the structure

$$-X-\overset{|}{\underset{|}{C}}-CH_2-S-,$$

this effect being distinguished from the hydrophobic adsorption.

This interaction may be called thiophilic ("attracted to sulfur"), said adsorption thus being a thiophilic adsorption.

R may be an unsubstituted alkyl group $CH_3(CH_2)_n-$. If n is a large integer there are obtained elements of hydrophobic as well as thiophilic adsorption where the hydrophobic adsorption tends to become predominant along with the increasing n. For n = o, i.e. R = methyl, there is practically obtained nothing but a thiophilic adsorption.

Consequently, the total adsorption effect can be modified in response to the chemical character of R. If a carboxyl group is introduced, the thiophilic adsorption is damped and the charge-dependent adsorption tends to take over.

The R group can be aromatic, e.g. phenyl, unsubstituted or substituted (Example 6). Strangely enough, the adsorption of proteins to a product according to the invention, having the structure

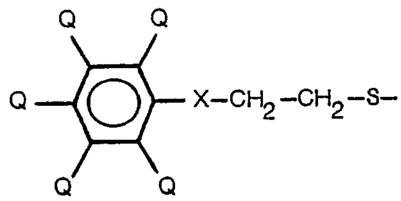 (IV)

wherein Q is H, OH or SH and with X containing S or Se, is most markedly distinguished from e.g. analogous adsorbents where X is —O— or —NH—.

The R group can be a heterocyclic group directly or indirectly bound to X, for example

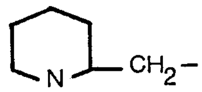 (V)

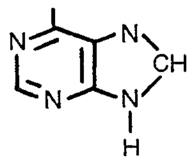 (VI)

The heterocyclic R groups may also comprise thenyl;

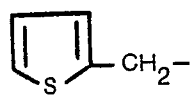 (VII)

although thenyl may be directly attached to a thiol group in the matrix:

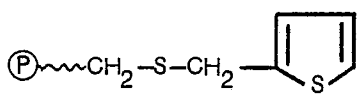 (VIII)

The adsorption is modified in various manners by the heterocyclic groups, but the thiophilic adsorption, manifested by the dependency on salt, will always remain.

Adsorbents according to the invention can be prepared from a thiol- or epoxide-containing carrier:

A) 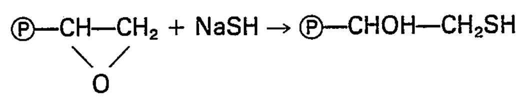 (IX)

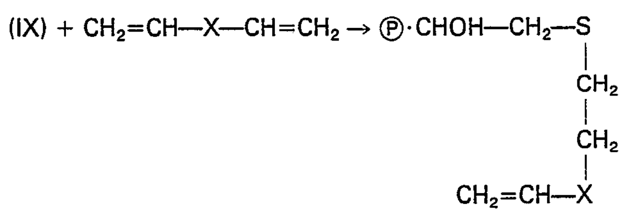 (X)

(X) + HS—$CH_2$—$CH_2OH$ →

Ⓟ—CHOH—$CH_2$—S—$CH_2$—$CH_2$—X—$CH_2$—$CH_2$—S—$CH_2$—$CH_2OH$ (XI)

B) Ⓟ—OH + $CH_2$=CH—X—CH=$CH_2$ → Ⓟ—O—$CH_2$—$CH_2$—X—CH=$CH_2$ (XII)

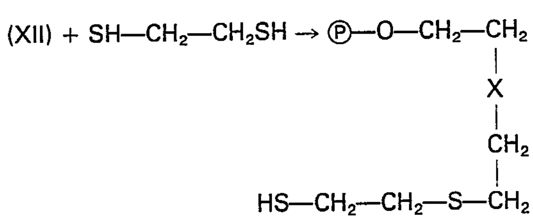 (XIII)

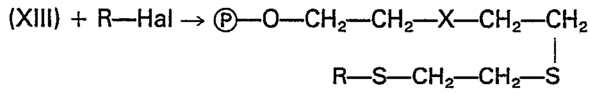

(XIV)

C) 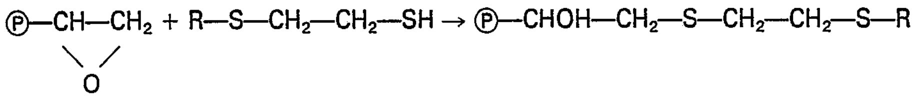 (XV)

D)

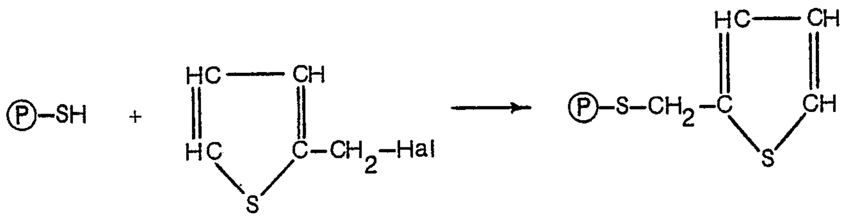

(XVI)

It is also possible in principle to prepare the inventive adsorbents directly from a hydroxyl- or amino group-containing carrier, for example

$$\text{Ⓟ—OH} + CH_2{=}CH\text{—}X\text{—}CH_2\text{—}CH_2\text{—}S\text{—}CH_2\text{—}CH_2\text{—}X\text{—}R \rightarrow$$
$$\text{Ⓟ—O—}CH_2\text{—}CH_2\text{—}X\text{—}CH_2\text{—}CH_2\text{—}S\text{—}CH_2\text{—}CH_2\text{—}X\text{—}R \quad \text{(XVII)}$$

It appears from the aforegoing that one embodiment of the invention can consist of particles constituting a netting for proteins which is permeable all the way to the center. In accordance with another alternative, only the surface layer of the particle is permeable for proteins, and in such a case the particle nucleus does not have to contain the group which is characteristic for adsorption. A third alternative can consist of a more or less heavy layer of netting surrounding an impermeable nucleus. The nucleus can be arbitrarily formed and constitute e.g. a fiber, the interior surface of a hose, a beaker or some other vessel, etc.

For the permeation of macromolecules and for the appropriate appearance of the distinguishing adsorption characteristics, the polymeric netting must have a specific property; it must be hydrophilic and permeable for the macromolecules, it must be resistant within a pH range where proteins can be adsorbed without being damaged, preferably within the range of pH 4—8 but suitably within a wider range. The chemical properties of the polymeric netting must also be well adapted to allow introduction of the group characteristic for the adsorption. With regard to the polymeric netting, the invention is restricted by these limitations to hydrophilic polymers of the following types: polyalcohols such as e.g. polyvinyl alcohols, polyhydroxy methanes, polysaccharides, polygalactane cross-linked hexitholes and polyamides such as cross-linked polyacryl amide and derivatives thereof. Also cross-linked polyamines and polyacides can be mentioned, although it should then be observed that the adsorption will be more complicated by the presence of the ionogenic groups. The gel netting can furthermore consist of inorganic gels to which have been coupled organic hydroxyl-containing substituents, for example substituted silicic acid gels, or groups such as

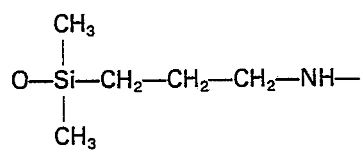

A particularly useful adsorbent according to the invention consists of cross-linked agar.

Example 1

Particulate 6% agarose gel was treated in a reaction vessel with 5% butanediol-bis-glycidyl ether in 0.6 M NaOH for 2 hours at 40°C. The gel was washed and then converted to thiol derivative by treatment with NaSH in 1 M $Na_2CO_3$ in the presence of 0.1% $NaBH_4$. The gel, "thiol agarose", was washed with distilled water followed by 0.1 M $NaHCO_3$, and was transferred to a reaction vessel for treatment during 1 hour with 1% divinyl sulfone. The divinyl sulfone may be added as such or as the precursor vinyl-β-halo-ethylsulfone. To the thiol agarose, treated with divinyl sulfone, was added excess mercapto ethanol and the mixture was allowed to react for 2 hours in 0.1 M $NaHCO_3$.

The gel was tested with regard to its ability of absorbing proteins. A gel bed of 5 ml was prepared and washed with 0.1 M Tris-HCl, tris-hydroxy methylamine methane-hydrochloride, 0.5 M $K_2SO_4$, at pH 7.6. Human serum dialyzed against this buffer solution to an amount of 50 absorbency properties (280 nm) was introduced into the bed, whereafter the gel was washed with 50 ml of buffer salt solution. Adsorbed protein was thereafter desorbed by first washing with 0.1 M tris-HCl, pH 7.6 (without $K_2SO_4$) and then with 40%

ethylene glycol, with 30% isopropanol, and finally with 95% ethanol. 45% protein was adsorbed to the gel of which 25% was eluated with the sulfate-free buffer and the remaining part with other eluents.

It was shown by electrophoretic analysis that all serum albumine passed the gel without adsorption, whereas immunoglobulin was fixed to the gel and was eluated bny the salt-free buffer. $\alpha_2$-macroglobuline as well as lipoproteins were also completely adsorbed.

Example 2

Example 1 was repeated but with the difference that cellulose powder was used instead of agarose particles.

Example 3

A trial was carried out with epichlorohydrin-treated polyacryl amide (commercial Eupergite). The oxirane gel was converted to SH-gel with NaSH, which was then treated with divinyl sulfone and thereafter with mercapto ethanol. The polyacrylamide derivative had a somewhat lower capacity than had the agarose gel according to Example 1, but the selectivity was principally the same.

Example 4

Thiol agarose was activated with divinyl sulfone followed by the attachment of 2.3-dihydroxy-propane-1-thiol as described in Example 1. In an adsorption test with human serum, 43% of the proteins having the same selectivity pattern as in Examples 1—3 was adsorbed but with the difference that the desorption with ethylene glycol was more efficient (10% compared to 6% in Example 1).

Example 5

The same procedure was employed as in Example 4 but with the use of 2.3-dimercapto-1-propanol. The adsorption capacity was lower (32%).

Example 6

Epichlorohydrin-activated agarose was converted into thiol gel and was treated with phenylvinyl sulfone in 0.1 M $NaHCO_3$. 20% serum protein was adsorbed on the gel, mainly by thiophilic adsorption as it was obvious that all albumine passed the bed without adhering.

Example 7

Thiolgel according to previous example was converted as described in Example 6 but with the difference that the phenylvinyl sulfone was exchanged for phenyl vinyl sulfoxide. The capacity was somewhat higher as compared with corresponding sulfone derivative (22%), although the selectivity was the same.

Example 8

Thiolagarose was divinylsulfone-activated as in Example 1 and was then converted with thioglycolic acid in 0.1 M $NaHCO_3$. The product was found to adsorb less than 5% protein. After esterification, the capacity was increased to 37% although the yield at desorption was low; total yield about 75%.

Example 9

Divinylsulfone-activated thiolagarose was converted with selenoglycolic acid analogously with Example 8. The adsorption capacity was low both prior to and after esterification. Reduction with $NaBH_4$ in LiCl solution resulted in an extremely high improvement of the adsorption capacity (48% increase). It proved very difficult however to desorb adhered protein (total yield 63%).

Example 10

Divinylsulfone-activated thiolagarose was linked with 2.2'-diethylamino ethane thiol in 0.1 M $NaHCO_3$. The product obtained was found to have an extremely low adsorption capacity (13%). Liptoproteins in principle were fixed to the gel.

Example 11

Agarose was activated with tosyl chloride in alkali and was then treated with 1.2-ethane thiol followed by methyl iodide. The product was found to have good capacity (43%). 36% of the adsorbed material was desorbed by the sulfate-free buffer. The total yield was quantitative.

**Claims**

1. Adsorbent intended for the thiophilic separation and immobilization of proteins, consisting of a carrier with covalently bound ligands, wherein the carrier constitutes a solid phase which is completely or partially penetrated by or surface-coated with a hydrophilic, molecular polymeric netting, characterized in that the bound ligands consist of the groups

$$R—X—CH_2—CH_2—Y—$$

wherein

y = S, $NR_1$ with $R_1$ being hydrogen or alkyl and either

i) X = $SO_2$, SO, S or Se;

R is $H—(CHQ)_n—(CH_2)_m—$ where

Q = H, OH, SH;

n = 0, 1, 2; and

m = 0, 1; or

R is an alkyl, hydroxy alkyl, mercapto alkyl, a phenyl group containing a nitro, hydroxyl or amino group (unsubstituted or substituted with alkyl, hydroxy alkyl or acyl), in ortho- or para-position to a thioether atom, or a heterocyclic, nitrogen-containing ring; with the exception that when RX = SH or $SO_3H$, Y is not S or NH; or

(ii) RX is formed of a heterocyclic ring

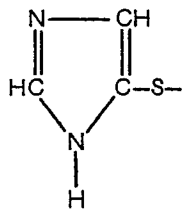

or

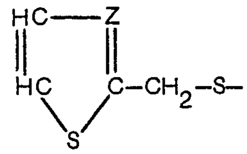

where Z = N or CH.

2. Adsorbent as claimed in Claims 1 and 2, characterized in that the polymeric netting is formed of a polyhydroxy polymer.

3. Adsorbent as claimed in any one of Claims 1—3, characterized in that the polyhydroxy polymer is a polygalactane, for example agar or agarose.

4. Adsorbent as claimed in Claim 1 or 2, characterized in that the polymeric netting is formed of a polyamide, for example cross-linked polyacryl amide or a derivative thereof.

5. Method of preparing an absorbent as claimed in any one of Claims 1—5, characterized in that thiol groups are first introduced into the polymeric netting for subsequent alkylization with vinyl-β-haloethyl sulfone, -sulfoxide or -sulfide.

6. Utilization of the adsorbent as claimed in Claim 1 for biopolymeric fractionation.

**Patentansprüche**

1. Adsorbens für die thiophile Trennung und Inmobilisierung von Proteinen, das aus einem Träger mit kovalent gebundenen Liganden besteht, wobei von dem Träger eine Festphase gebildet wird, die vollständig oder teilweise durchdrungen ist von, oder oberflächenbeschichtet ist mit einem hydrophilen, molekular polymeren Netz, dadurch gekennzeichnet, daß die gebundenen Liganden aus den Gruppen bestehen:

$$R—X—CH_2—CH_2—Y—$$

in der Y = S, $NR_1$ mit $R_1$ = Wasserstoff oder Alkyl, und entweder

i) X = $SO_2$, SO, S oder Se;

R = $H—(CHQ)_n—(CH_2)_m—$ worin

Q = H, OH, SH;

n = 0, 1, 2 und

m = 0, 1; oder

R ein Alkyl, Hydroxyalkyl, Mercaptoalkyl, eine Phenylgruppe, die eine Nitro-, Hydroxyl- oder Aminogruppe (unsubstituiert oder substituiert mit Alkyl, Hydroxyalkyl oder Acyl) in ortho- oder para-Stellung zu einem Thioetheratom oder einem heterozyklischen, Stickstoff enthaltenden Ring enthält; mit der Ausnahme, daß wenn RX = SH oder $SO_3H$ bedeutet, Y nicht S oder NH ist, oder

ii) RX von einem heterozyklischen Ring

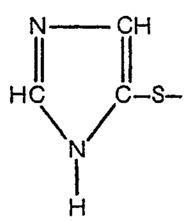

oder

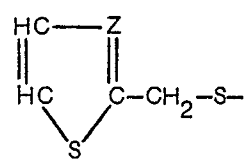

gebildet wird, worin Z = N oder CH bedeutet.

2. Adsorbens gemäß Anspruch 1, dadurch gekennzeichnet, daß das polymere Netz aus einem Polyhydroxypolymer gebildet wird.

3. Adsorbens gemäß einem der Ansprüche 1 bis 2, dadurch gekennzeichnet, daß das Polyhydroxypolymer ein Polygalaktan ist, zum Beispiel Agar oder Agarose.

4. Adsorbens gemäß Anspruch 1, dadurch gekennzeichnet, daß das polymere Netz aus einem Polyamid gebildet wird, zum Beispiel einem vernetzten Polyacrylamid oder einem Derivat davon.

5. Verfahren zur Herstellung eines Adsorbens gemäß einem der Ansprüche 1 bis 4, dadurch gekennzeichnet, daß Thiolgruppen zuerst in das polymere Netz eingeführt werden zur nachfolgenden Alkylierung mit Vinyl-β-haloethylsulfon, -sulfoxid oder -sulfid.

6. Verwendung des Adsorbens gemäß dem Anspruch 1 zur biopolymeren Fraktionierung.

**Revendications**

1. Adsorbant destiné à la séparation et à l'immobilisation thiophiles de protéines, constitué d'un support avec des ligands liés par covalence, dans lequel le support constitue une phase solide qui est complètement ou partiellement pénétrée, ou revêtue en surface, par un réseau polymère moléculaire hydrophile, caractérisé en ce que les ligands liés consistent en les groupes

$$R—X—CH_2—CH_2—Y—$$

où

Y = S, $NR_1$, $R_1$ étant un hydrogène ou un alkyle et soit

i) X = $SO_2$, SO, S ou Se;

R est $H—(CHQ)_n—(CH_2)_m—$ où

Q = H, OH, SH;

n = 0, 1 ou 2; et

m = 0 ou 1;

ou R est un alkyle, un hydroxyalkyle, un mercaptoalkyle, un groupe phényle contenant un groupe nitro, hydroxyle ou amino (non substitué ou substitué par un alkyle, un hydroxyalkyle ou un acyle) en position ortho ou para relativement à un atome de thioéther, ou un hétérocycle azoté;

si ce n'est que lorsque RX = SH ou $SO_3H$, Y n'est ni S ni NH, soit

ii) RX est formé d'un hétérocycle

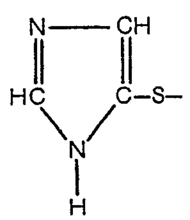 ou 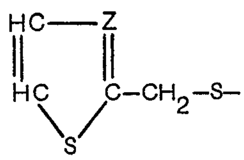

où Z = N ou CH.

2. Adsorbant selon la revendication 1, caractérisé en ce que le réseau polymère est formé d'un polymère polyhydroxylé.

3. Adsorbant selon une des revendications 1 ou 2, caractérisé en ce que le polymère hydroxylé est un polygalactane, par exemple l'agar ou l'agarose.

4. Adsorbant selon la revendication 1, caractérisé en ce que le réseau polymère est formé d'un polyamide, par exemple un polyacrylamide réticulé ou un dérivé de celui-ci.

5. Procédé pour préparer un adsorbant selon l'une quelconque des revendications 1 à 4, caractérisé en ce qu'on introduit tout d'abord des groupes thiols dans le réseau polymère pour effectuer ultérieurement une alkylation avec une sulfone, un sulfoxyde ou un sulfure vinyl-β-halogénoéthyliques.

6. Utilisation de l'adsorbant selon la revendication 1 pour le fractionnement d'un biopolymère.